# EUROPÄISCHE PATENTSCHRIFT

(11) **EP 0 485 894 B1**
(45) Veröffentlichungstag und Bekanntmachung des Hinweises auf die Patenterteilung: **03.02.1999**
(21) Anmeldenummer: 91119006.4
(22) Anmeldetag: 07.11.1991
(51) Int. Cl.: C07H 15/252, C07H 15/26, A61K 31/70

(54) **Verfahren zur stereoselektiven Herstellung von semisynthetischen diastereomerenreinen N-Glycidyl-Anthracyclinen**
Methods for the preparation of semi-synthetic diastereomerically pure N-glycidyl-anthracyclines
Méthode de préparation de diastereoisomeriquement pur N-glycidyl-anthracyclines semi-synthétiques

(30) Priorität: 14.11.1990 DE 4036155
(43) Veröffentlichungstag der Anmeldung: 20.05.1992
(73) Patentinhaber: HOECHST AKTIENGESELLSCHAFT, 65929 Frankfurt am Main (DE)
(72) Erfinder: Gerken, Manfred, Dr., W-3550 Marburg (DE); Grimm, Monika, W-6301 Biebertal (DE); Raab, Ernst, W-3550 Marburg (DE); Hoffmann, Dieter, Dr., W-3550 Marburg (DE); Straub, Reiner, W-3550 Marburg (DE)
(74) Vertreter: Then, Johann, Dr.

(56) Entgegenhaltungen:
- EP-A- 0 345 598
- JOURNAL OF ORGANIC CHEMISTRY Bd. 51, Nr. 19, 19. September 1986, WASHINGTON D.C. (US) Seiten 3710 - 3712; J.M.KLUNDER, S.Y.KO, AND K. BARRY SHARPLESS: 'ASSYMETRIC EPOXIDATION OF ALLYL ALCOHOL: EFFICIENT ROUTES TO HOMOCHIRAL BETA-ADRENERGIC BLOCKING AGENTS'

## Beschreibung

Die vorliegende Erfindung betrifft ein Verfahren zur Herstellung zytostatisch wirksamer N-(R)-glycidyl-Anthracycline und N-(S)-glycidyl-Anthracycline der Formel I 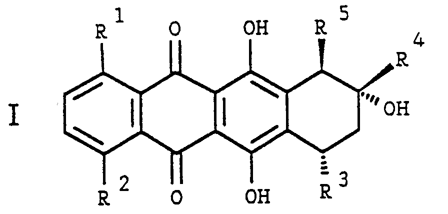 für welche gilt
- R¹: ist Wasserstoff oder eine Hydroxygruppe,
- R²: ist Wasserstoff, eine Hydroxygruppe oder eine Alkyloxygruppe (C1-C4),
- R³: ist Wasserstoff, eine Hydroxygruppe oder eine Struktur der Formel II, 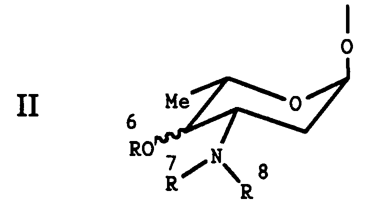
- R⁴: ist CH₂CH₃, COCH₃, COCH₂OH, CHOHCH₃ oder CHOHCH₂OH,
- R⁵: ist Wasserstoff, eine Hydroxygruppe, eine Methoxycarbonylgruppe oder eine Struktur der Formel II, wobei entweder R³ oder R⁵ oder beide eine Struktur der Formel II sein muß,
- R⁶: ist Wasserstoff, eine Alkylgruppe (C1-C4), eine Allylgruppe, eine Benzylgruppe oder mono- oder di-methyloxy-substituierte Benzylgruppe, eine Tetrahydropyranylgruppe,
- R⁷: ist Wasserstoff, eine Alkylgruppe (C1-C4), eine Allylgruppe, eine Benzylgruppe oder mono- oder di-methyloxy-substituierte Benzylgruppe und
- R⁸: ist eine Struktur der Formel III oder IV, 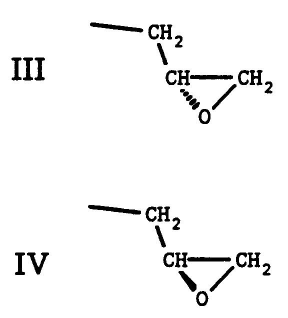
besonders von 7-O-(3-N-Methyl-3-N-(R)-glycidyl-α-L-daunosaminyl)-β-rhodomycinon und 7-O-(3-N-Methyl-3-N-(S)-glycidyl-α-L-daunosaminyl)-β-rhodomycinon, die aufgrund ihrer zytostatischen Wirksamkeit für die Behandlung von Krebserkrankungen geeignet sind.

Die Herstellung von 7-O-(3-N-Methyl-3-N-(R/S)-glycidyl-α-L-daunosaminyl)-β-rhodomycinon und dessen zytotoxische Aktivität ist in der Patentanmeldung DE 3819092 A1 als stereounspezifische Synthese beschrieben. Dieses Verfahren geht von racemischen Epibromhydrin aus, welches an die N-Methyl-Aminogruppe des Daunosamins am Anthracyclin angelagert wird. Dabei werden die erfindungsgemäßen Verbindungen als Diastereomerengemisch erhalten, das sich nur unter mehrfachen aufwendigen chromatographischen Methoden sehr verlustreich trennen läßt. Hierbei zeigte sich, daß wegen der enormen Instabilität der Verbindungen die Chromatographie des R/S-Diastereomerengemisches die reinen Diastereomeren nicht in der nötigen Reinheit und der erforderlichen Menge liefern konnte, da ein großer Teil der Substanzmenge irreversibel an den Kieselgelträger während der Trennung gebunden wurde.

Aus US 4408063, J. Org. Chem. **43**, 4876-4878 (1978) und J. Org. Chem. **47**, 3581-3585 (1982) ist die Herstellung von R- und S-Epibromhydrin bekannt, wobei es sich um vielstufige aufwendige Synthesen handelt, die bei der Reproduktion nur unzureichende Ausbeuten in einer ungenügenden optischen Reinheit an R- bzw. S-Epibromhydrin liefern.

Es wurde völlig überraschend festgestellt, daß bei der Umsetzung von 7-O-(3-N-Methyl-α-L-daunosaminyl)-β-rhodomycinon (DE 3641833 A1) mit einem Zwischenprodukt aus der Epibromhydrinsynthese, nämlich dem 3-Bromo-1-tosyloxy-2-(R)-propanol (J. Org. Chem. **47**, 3581-3585 (1982)) direkt das 7-O-(3-N-Methyl-3-N-(S)-glycidyl-α-L-daunosaminyl)-β-rhodomycinon erhalten wird.

Weiterhin wurde überraschenderweise festgestellt, daß sich das durch Sharpless-Epoxydierung und anschließender Tosylierung aus Allylalkohol leicht und sehr enantiomerenrein herstellbare (R)-Glycidyl-tosylat und (S)-Glycidyl-tosylat (J. Org. Chem. **51**, 3710-3712 (1986)) ebenfalls mit 7-O-(3-N-Methyl-α-L-daunosaminyl)-β-rhodomycinon zum 7-O-(3-N-Methyl-3-N-(S)-glycidyl-α-L-daunosaminyl)-β-rhodomycinon umsetzen läßt.

Überraschenderweise konnte die chromatographische Reinigung der Reaktionsprodukte an, mit saurem wässrigem Puffer vorbehandeltem und desaktiviertem Kieselgel durchgeführt werden, ohne daß größere Substanzmengen irreversibel an das Kieselgel binden.

Ausgehend von diesem Stand der Technik liegt der Erfindung die Aufgabe zugrunde, ein neues Verfahren zu entwickeln, das N-(R)-glycidyl-Anthracycline und N-(S)-glycidyl-Anthracycline in einer stereoselektiven Synthese in guten Ausbeuten und in einer Reinheit über 96% liefert und das eine Vereinfachung gegenüber bekannten Verfahren bedeutet.

Diese Aufgabe wird erfindungsgemäß gelöst durch das Verfahren zur Herstellung von N-(R)-glycidyl-Anthracyclinen oder N-(S)-glycidyl-Anthracyclinen der Formel I 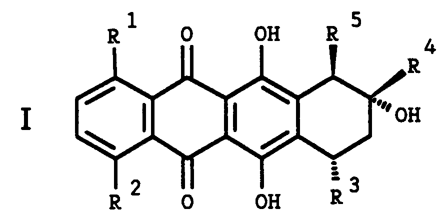 für welche gilt
- R¹: ist Wasserstoff oder eine Hydroxygruppe,
- R²: ist Wasserstoff, eine Hydroxygruppe oder eine Alkyloxygruppe (C1-C4),
- R³: ist Wasserstoff, eine Hydroxygruppe oder eine Struktur der Formel II, 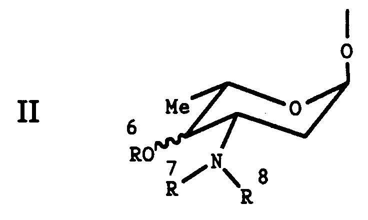
- R⁴: ist CH₂CH₃, COCH₃, COCH₂OH, CHOHCH₃ oder CHOHCH₂OH,
- R⁵: ist Wasserstoff, eine Hydroxygruppe, eine Methoxycarbonylgruppe oder eine Struktur der Formel II, wobei entweder R³ oder R⁵ oder beide, eine Struktur der Formel II sein muß,
- R⁶: ist Wasserstoff, eine Alkylgruppe (C1-C4), eine Allylgruppe, eine Benzylgruppe oder mono- oder di-methyloxy-substituierte Benzylgruppe, eine Tetrahydropyranylgruppe,
- R⁷: ist Wasserstoff, eine Alkylgruppe (C1-C4), eine Allylgruppe, eine Benzylgruppe oder mono- oder di-methyloxy-substituierte Benzylgruppe und
- R⁸: ist eine Struktur der Formel III oder IV, 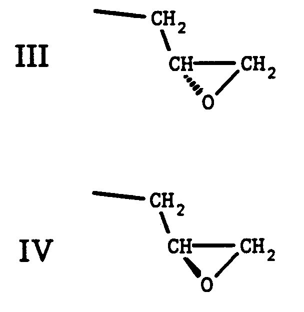
dadurch gekennzeichnet,
daß man ein Anthracyclin-Derivat der Struktur I, in der
- R¹, R² und R⁴: wie oben definiert ist und
- R³: ist Wasserstoff, eine Hydroxygruppe oder eine Struktur der Formel V, 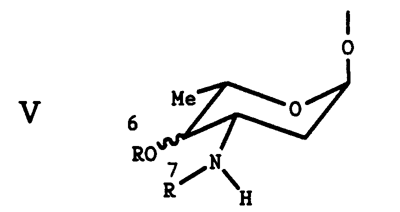
R⁵ ist Wasserstoff, eine Hydroxygruppe, eine Methoxycarbonylgruppe oder eine Struktur der Formel V, wobei entweder R³ oder R⁵ oder beide eine Struktur der Formel V sein muß, und
R⁶ und R⁷ wie oben definiert ist,
mit einem (R)- oder (S)-Glycidyl-sulfonat, beispielsweise (R)- oder (S)-Glycidyl-tosylat, (R)- oder (S)-Glycidyl-mesylat, (R)- oder (S)-Glycidyl-brosylat oder (R)- oder (S)-Glycidyl-trifluormethansulfonat umsetzt und das Produkt gewinnt.

Dazu kann man in Gegenwart einer Base, beispielsweise Kaliumcarbonat, Triethylamin oder Pyridin, und in einem geeigneten organischen Lösungsmittel oder Lösungsmittelgemisch, beispielsweise N,N-Dimethylformamid oder Acetonitril bei 20° C bis zur Rückflußtemperatur des Lösungsmittels 1 bis 48 Stunden rühren und die Reaktionlösung aufarbeiten. Es kann aufgearbeitet werden, indem man aus der Lösung, gegebenenfalls nach Neutralisation, das Rohprodukt durch Einengen oder Extraktion gewinnt und gegebenenfalls reinigt, vorzugsweise durch chromatographische Trennung an, mit einer wässrigen Pufferlösung desaktiviertem Kieselgel, wobei man das Produkt der Formel I in einer Reinheit größer als 96 % erhält.

Bevorzugt werden nach dem erfindungsgemaßen Verfahren Verbindungen der Formel I hergestellt, worin
- R¹: eine Hydroxygruppe,
- R²: eine Hydroxygruppe oder eine Methyloxygruppe,
- R³: eine Struktur der Formel II, 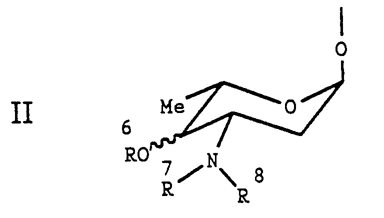
- R⁴: CH₂CH₃, COCH₃, COCH₂OH,
- R⁵: Wasserstoff, eine Hydroxygruppe oder eine Methoxycarbonylgruppe,
- R⁶: Wasserstoff oder eine Tetrahydropyranylgruppe,
- R⁷: Wasserstoff oder Methyl,
- R⁸: eine Struktur der Formel III oder IV, 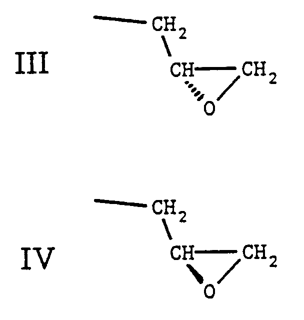
bedeuten.
- R¹: ist Wasserstoff oder eine Hydroxygruppe,
- R²: ist Wasserstoff, eine Hydroxygruppe oder eine Alkyloxygruppe (C1-C4),
- R³: ist Wasserstoff, eine Hydroxygruppe oder eine Struktur der Formel II,
- R⁴: ist CH₂CH₃, COCH₃, COCH₂OH, CHOHCH₃ oder CHOHCH₂OH,
- R⁵: ist Wasserstoff, eine Hydroxygruppe, eine Methoxycarbonylgruppe oder eine Struktur der Formel II, wobei entweder R³ oder R⁵ oder beide eine Struktur der Formel II sein muß,
- R⁶: ist Wasserstoff, eine Alkylgruppe (C1-C4), eine Allylgruppe, eine Benzylgruppe oder mono- oder di-methyl-oxy-substituierte Benzylgruppe, eine Tetrahydropyranylgruppe,
- R⁷: ist Wasserstoff, eine Alkylgruppe (C1-C4), eine Allylgruppe, eine Benzylgruppe oder mono- oder di-methyl-oxy-substituierte Benzylgruppe und
- R⁸: ist eine Struktur der Formel III oder IV.

Bevorzugt sind:
N-Glycidyl-Anthracyclinderivate der Formel I, worin
- R¹: ist Wasserstoff oder eine Hydroxygruppe,
- R²: ist Wasserstoff, eine Hydroxygruppe oder eine Methoxygruppe,
- R³: ist eine Struktur der Formel II,
- R⁴: ist CH₂CH₃, COCH₃, COCH₂OH, CHOHCH₃ oder CHOHCH₂OH,
- R⁵: ist Wasserstoff, eine Hydroxygruppe, eine Methoxy carbonylgruppe oder eine Struktur der Formel II,
- R⁶: ist Wasserstoff, eine Alkylgruppe (C1-C4), eine Allylgruppe, eine Benzylgruppe oder mono- oder di-methyl-oxy-substituierte Benzylgruppe, eine Tetrahydropyranylgruppe,
- R⁷: ist Wasserstoff, eine Alkylgruppe (C1-C4), eine Allylgruppe, eine Benzylgruppe oder mono- oder di-methyl-oxy-substituierte Benzylgruppe und
- R⁸: ist eine Struktur der Formel III oder IV ;

N-Glycidyl-Anthracyclinderivate der Formel I, worin
- R¹: ist Wasserstoff oder eine Hydroxygruppe,
- R²: ist Wasserstoff, eine Hydroxygruppe oder eine Methoxygruppe,
- R³: ist eine Struktur der Formel II,
- R⁴: ist CH₂CH₃, COCH₃, COCH₂OH, CHOHCH₃ oder CHOHCH₂OH,
- R⁵: ist Wasserstoff, eine Hydroxygruppe, eine Methoxy carbonylgruppe,
- R⁶: ist Wasserstoff, eine Alkylgruppe (C1-C4), eine Allylgruppe, eine Benzylgruppe oder mono- oder di-methyl-oxy-substituierte Benzylgruppe, eine Tetrahydropyranylgruppe,
- R⁷: ist Wasserstoff, eine Alkylgruppe (C1-C4), eine Allylgruppe, eine Benzylgruppe oder mono- oder di-methyl-oxy-substituierte Benzylgruppe und
- R⁸: ist eine Struktur der Formel III oder IV;

N-Glycidyl-Anthracyclinderivate der Formel I, worin
- R¹: ist Wasserstoff,
- R²: ist Wasserstoff, eine Hydroxygruppe oder eine Methoxygruppe,
- R³: ist eine Struktur der Formel II,
- R⁴: ist CH₂CH₃, COCH₃, COCH₂OH, CHOHCH₃ oder CHOHCH₂OH,
- R⁵: ist Wasserstoff, eine Hydroxygruppe, eine Methoxy carbonylgruppe,
- R⁶: ist Wasserstoff, eine Tetrahydropyranylgruppe,
- R⁷: ist Wasserstoff, eine Alkylgruppe (C1-C4), eine Allylgruppe, eine Benzylgruppe oder mono- oder di-methyl-oxy-substituierte Benzylgruppe und
- R⁸: ist eine Struktur der Formel III oder IV;

N-Glycidyl-Anthracyclinderivate der Formel I, worin
- R¹: ist Wasserstoff,
- R²: ist Wasserstoff, eine Hydroxygruppe oder eine Methoxygruppe,
- R³: ist eine Struktur der Formel II,
- R⁴: ist CH₂CH₃, COCH₃, COCH₂OH, CHOHCH₃ oder CHOHCH₂OH,
- R⁵: ist Wasserstoff, eine Hydroxygruppe, eine Methoxy carbonylgruppe,
- R⁶: ist Wasserstoff, eine Tetrahydropyranylgruppe,
- R⁷: ist Wasserstoff, eine Alkylgruppe (C1-C4), eine Allylgruppe und
- R⁸: ist eine Struktur der Formel III oder IV;

N-Glycidyl-Anthracyclinderivate der Formel I, worin
- R¹: ist Wasserstoff,
- R²: ist eine Hydroxygruppe oder eine Methoxygruppe,
- R³: ist eine Struktur der Formel II,
- R⁴: ist CH₂CH₃, COCH₃ oder COCH₂OH,
- R⁵: ist Wasserstoff, eine Hydroxygruppe,
- R⁶: ist Wasserstoff,
- R⁷: ist Wasserstoff, eine Alkylgruppe (C1-C4), eine Allylgruppe und
- R⁸: ist eine Struktur der Formel III oder IV;

N-Glycidyl-Anthracyclinderivate der Formel I, worin
- R¹: ist Wasserstoff,
- R²: ist eine Hydroxygruppe,
- R³: ist eine Struktur der Formel II,
- R⁴: ist CH₂CH₃,
- R⁵: ist eine Hydroxygruppe,
- R⁶: ist Wasserstoff,
- R⁷: ist eine Methylgruppe und
- R⁸: ist eine Struktur der Formel III oder IV;

N-Glycidyl-Anthracyclinderivate der Formel I, worin
- R¹: ist Wasserstoff,
- R²: ist eine Hydroxygruppe,
- R³: ist eine Struktur der Formel II,
- R⁴: ist CH₂CH₃,
- R⁵: ist eine Hydroxygruppe,
- R⁶: ist Wasserstoff,
- R⁷: ist eine Methylgruppe und
- R⁸: ist eine Struktur der Formel III.

Die folgenden Beispiele erläutern die Erfindung näher, ohne sie zu beschränken:

### Beispiel 1:

### 7-O-(3-N-Methyl-3-N-(R)-glycidyl-α-L-daunosaminyl)-β-rhodomycinon

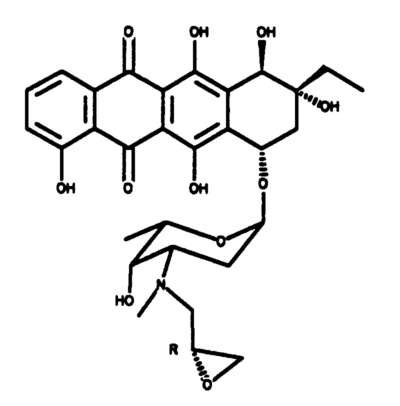

Zu einer Lösung von 7-O-(3-N-Methyl-α-L-daunosaminyl)-β-rhodomycinon (1.25g, 2.36 mmol) in trockenem N,N-Dimethylformamid (20 ml) werden nacheinander (R)-Glycidyl-tosylat (2.7 g, 11.83 mmol) und Kaliumcarbonat (3.2 g, 23.9 mmol) gegeben und die Suspension wird 2.5 h bei 85° C gerührt. Nach Abkühlen auf Raumtemperatur wird die Reaktionslösung in Wasser (300 ml) gegossen, mit konz. Essigsäure neutralisiert und mit Chloroform (5 X 50 ml) extrahiert. Die vereinigten organischen Extrakte werden nochmals mit Wasser (100 ml) gewaschen und am Hochvakuum bei niedriger Temperatur konzentriert. Die Substanz wird säulenchromatographisch (Kieselgel 60, 0.040-0.063 mm, Merck Nr. 9385; desaktiviert durch Rühren mit wässrigem Triethylamin / Phosphat-Puffer pH 3 und anschliessendem Trockensaugen) vorgereinigt (Laufmittel: Dichlormethan / Isopropanol / Acetonitril 80 / 12 / 8). Durch Mitteldruckchromatographie (Kieselgel Macherey & Nagel Nucleosil 100-1525; desaktiviert durch Rühren mit wässrigem Triethylamin / Phosphat-Puffer pH 3 und anschliessendem Trockensaugen) wird die Endreinigung vorgenommen (Laufmittel: Dichlormethan / Isopropanol / Acetonitril 80 / 12 / 8).
Ausbeute: 150 mg (11 %); Reinheit >98 %.

Physikalische Daten:
Rf-Wert: 0.30 (Laufmittel: Dichlormethan / Isopropanol / Acetonitril 80 / 12 / 8).
Schmelzpunkt: 120° C (zersetzt).
[α]^{D}₂₀ = +335° (c = 0.02 in CHCl₃).
¹H-NMR (200 MHz in CDCl₃):
   δ 13.61 (s, 1H, OH-11), 12.83 (s, 1H, OH-6), 12.15 (s, 1H, OH-4), 7.88 (dd, 1H, J_{1,2} = 7.5 Hz, J_{1,3} = 1.1 Hz, H-1), 7.72 (t, 1H, J_{1,2} = J_{2,3} = 7.5 Hz, H-2), 7.32 (dd, 1H, J_{1,3} = 1.1 Hz, J_{2,3} = 7.5 Hz, H-3), 5.52 (bs, 1H, H-1'), 5.30 (dd, 1H, J_{7,8a} = 1.4 Hz, J_{7,8b} = 4.1 Hz, H-7), 4.90 (s, 1H, H-10), 4.09 (q, 1H, J_{5',6'} = 6.5 Hz, H-5'), 4.02 (s, 1H, OH), 3.72 (bs, 1H, H-4), 2.99 (m, 1H, H-2''), 2.79 (dd, 1H, J_{1a'',1b''} = -11.6 Hz, J_{1a'',2''} = 3.4 Hz, H-1a''), 2.75 (t, J_{3a'',3b''} = J_{2'',3a''} = 4.3 Hz, 1H, H-3a''), 2.55 (m, 1H, H-3'), 2.45 (dd, 1H, J_{2'',3b''} = 2.7 Hz, J_{3a'',3b''} = 4.9 Hz, H-3b''), 2.37 (dd, 1H, J_{1a'',1b''} = -11.6 Hz, J_{1b'',2''} = 3.1 Hz, H-1b''), 2.36 (s, 3H, NCH₃), 2.25 (dd, 1H, J_{7,8a} = 1.4 Hz, J_{8a,8b} = -11.0 Hz, H-8a), 2.15 (dd, 1H, J_{7,8b} = 4.1 Hz, J_{8a,8b} = -11.1 Hz, H-8b), 1.82 (m, 4H, H-13a, H-13b, H-2a', H-2e'), 1.41 (d, 3H, J_{5',6'} = 6.5 Hz, H-6'), 1.12 (t, 3H, J_{13,14} = 7.5 Hz, H-14) ppm.
¹³C-NMR (50 MHz in CDCl₃):
   δ 190.6 (C-5), 186.1 (C-12), 162.6 (C-4), 157.2 (C-11), 156.7 (C-6), 138.6 (C-10a), 137.1 (C-2), 134.9 (C-6a), 133.2 (C-12a), 124.9 (C-3), 119.7 (C-1), 115.9 (C-4a), 112.0 (C-5a), 111.4 (C-11a), 101.3 (C-1'), 71.8 (C-9), 70.8 (C-7), 66.7 (C-5'), 66.5 (C-10), 66.3 (C-4'), 57.9 (C-3'), 54.9 (C-1''), 50.3 (C-2''), 45.3 (C-3''), 39.1 (C-NCH₃), 32.8 (C-8), 30.3 (C-13), 28.5 (C-2'), 17.0 (C-6'), 6.6 (C-14) ppm.
Massenspektr.: FAB-MS *m/z* 586 (M⁺).
UV-Spektr.: λₘₐₓ^{MeOH} nm (ε) 230 (25,200), 292 (10,000), 494 (17,200), 528 (13,500), 587 (4,400).
IR-Spektr.: (KBr)cm⁻¹ 3440 (νₘₐₓ), 2964, 1600, 1460, 1437, 1405, 1291, 1237, 1198, 1165, 1130, 1067, 1022, 983.

Die Bestimmung der Konfiguration am Glycidylrest erfolgte folgendermaßen:
Die Öffnung des Epoxides der Verbindung aus Beispiel 1 im wässrigen sauren Milieu erfolgt gemäß der Literatur (z. B. Parker et al. Chem. Rev. 59, 737 (1959)) so, daß die am stärksten substituierte C-O Bindung gespalten wird und man über den Übergangszustand **3** das Diol **5** erhält. Unter den basischen Reaktionsbedingungen der Herstellung der Verbindung aus Beispiel 1 erhält man in geringen Mengen das Nebenprodukt **4**, welches durch basiche Epoxidöffnung mit Spaltung der am wenigsten substituierten C-O Bindung gemäß der Literatur (z. B. Parker et al. Chem. Rev. 59, 737 (1959)) entsteht. Dieses cyclische Carbonat kann durch Behandlung mit Natriummethanolat in das Diol **6** überführt werden.
Die Diole **5** und **6** wurden auf anderem Wege, gemäß dem Patent DE 3641833 A1 aus optisch reinem Glycerinaldehyd hergestellt und mit den aus der Epoxidöffnung erhaltenen Produkten mittels HPLC-Analytik verglichen.
Aus dem Entstehen der Produkte **5** und **6** muß gefolgert werden, das das Produkt aus Beispiel 1 am Glycidylrest R-konfiguriert vorliegt.

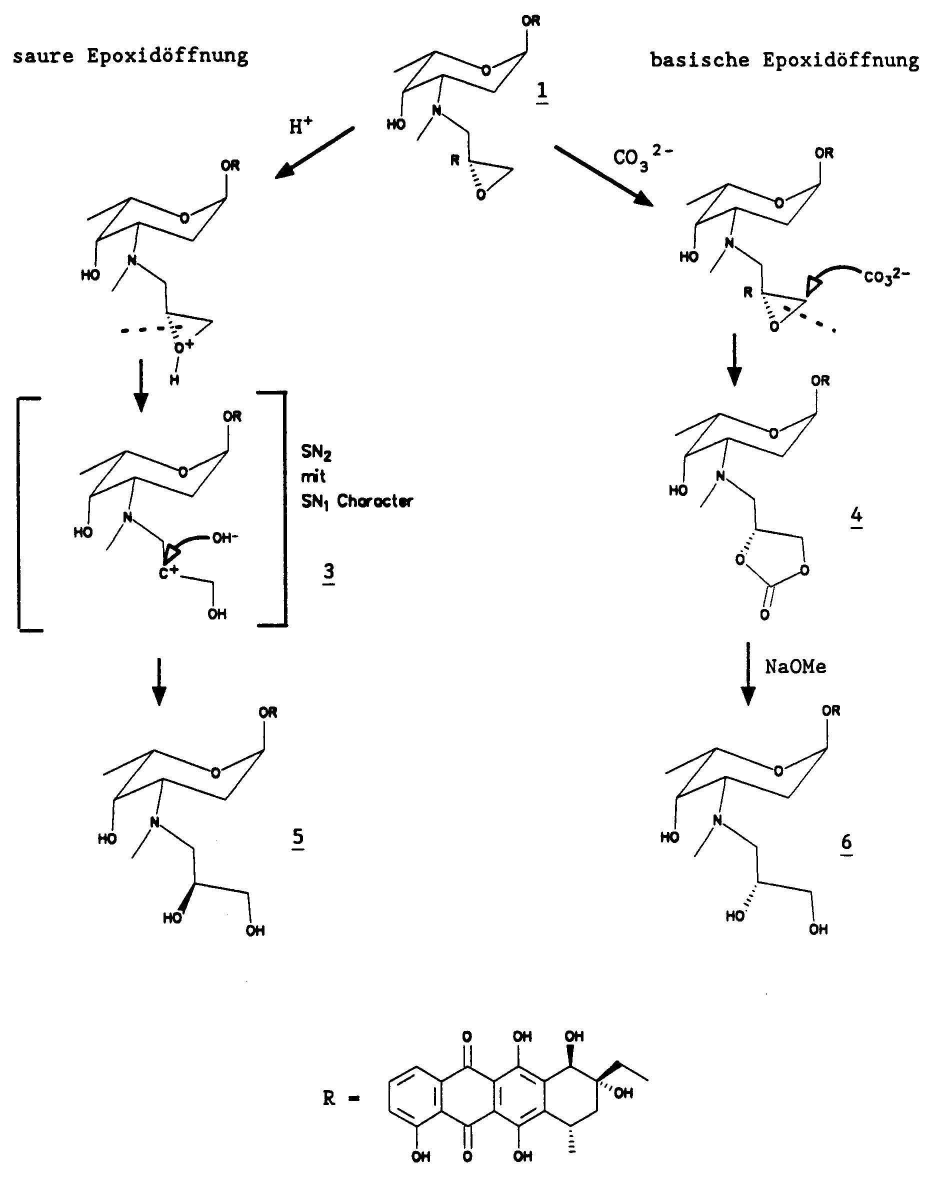

### Beispiel 2:

### 7-O-(3-N-Methyl-3-N-(S)-glycidyl-α-L-daunosaminyl)-β-rhodomycinon

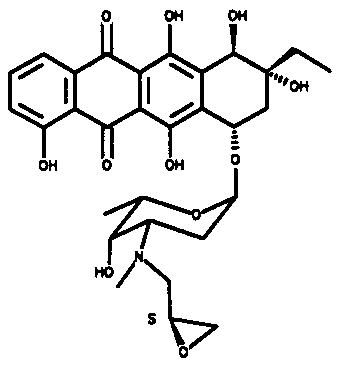

7-O-(3-N-Methyl-α-L-daunosaminyl)-β-rhodomycinon wurde entsprechend Beispiel 1 mit (S)-Glycidyl-tosylat umgesetzt, aufgearbeitet und gereinigt.

Physikalische Daten:
Rf-Wert: 0.35 (Laufmittel: Dichlormethan / Isopropanol /
Acetonitril 80 / 12 / 8).
Schmelzpunkt: 212-215° C.
[α]^{D}₂₀ = +187° (c = 0.075 in CHCl₃).
¹H-NMR (400 MHz in CDCl₃):
   δ 13.58 (s, 1H, OH-11), 12.81 (s, 1H, OH-6), 12.11 (s, 1H, OH-4), 7.87 (dd, 1H, J_{1,2} = 7.5 Hz, J_{1,3} = 1.1 Hz, H-1), 7.71 (t, 1H, J_{1,2} = J_{2,3} = 7.5 Hz, H-2), 7.31 (dd, 1H, J_{1,3} = 1.1 Hz, J_{2,3} = 7.5 Hz, H-3), 5.51 (bs, 1H, H-1'), 5.14 (dd, 1H, J_{7,8a} = 1.8 Hz, J_{7,8b} = 4.0 Hz, H-7), 4.90 (s, 1H, H-10), 4.08 (q, 1H, J_{5',6'} = 6.6 Hz, H-5'), 4.03 (s, 1H, OH), 3.69 (bs, 1H, H-4), 2.99 (m, 1H, H-2''), 2.95 (dd, 1H, J_{1a''1b''} = -14.4 Hz, J_{1a'',2''} = 2.8 Hz, H-1a''), 2.72 (dd, J_{3a'',3b''} = -4.8 Hz, J_{2'',3a''} = 4.1 Hz, 1H, H-3a''), 2.54 (m, 1H, H-3'), 2.49 (dd, 1H, J_{2'',3b''} = 2.3 Hz, J_{3a'',3b''} = 4.8 Hz, H-3b''), 2.32 (dd, 1H, J_{1a'',1b''} = -14.4 Hz, J_{1b'',2''} = 5.7 Hz, H-1b''), 2.31 (s, 3H, NCH₃), 2.24 (dd, 1H, J_{7,8a} = 1.8 Hz, J_{8a,8b} = -14.0 Hz, H-8a), 2.11 (dd, 1H, J_{7,8b} = 4.0 Hz, J_{8a,8b} = -14.0 Hz, H-8b), 1.82 (m, 4H, H-13a, H-13b, H-2a', H-2e'), 1.41 (d, 3H, J_{5',6'} = 6.6 Hz, H-6'), 1.12 (t, 3H, J_{13,14} = 7.5 Hz, H-14) ppm.
¹³C-NMR (100 MHz in CDCl₃):
   δ 190.5 (C-5), 186.0 (C-12), 162.5 (C-4), 157.0 (C-11), 156.6 (C-6), 138.4 (C-10a), 136.9 (C-2), 134.7 (C-6a), 133.0 (C-12a), 124.8 (C-3), 119.5 (C-1), 115.7 (C-4a), 111.9 (C-5a), 111.2 (C-11a), 101.1 (C-1'), 71.7 (C-9), 70.6 (C-7), 66.5 (C-5'), 66.4 (C-10), 66.0 (C-4'), 57.6 (C-3'), 54.4 (C-1''), 50.4 (C-2''), 44.5 (C-3''), 39.1 (C-NCH₃), 32.6 (C-8), 30.2 (C-13), 28.4 (C-2'), 16.8 (C-6'), 6.4 (C-14) ppm.
Massenspektr.: FAB-MS *m/z* 586 (M⁺).
UV-Spektr.: λₘₐₓ^{MeOH} nm (ε) 235 (62,800), 295 (13,900), 512 (18,500), 541 (21,700), 586 (17,400).
IR-Spektr.: (KBr)cm⁻¹ 3446 (νₘₐₓ), 2928, 1602, 1460, 1437, 1404, 1290, 1236, 1198, 1166, 1130, 1067, 1024, 982.

## Patentansprüche

1. Verfahren zur Herstellung von N-(R)-glycidyl-Anthracyclinen und N-(S)-glycidyl-Anthracyclinen der Formel I 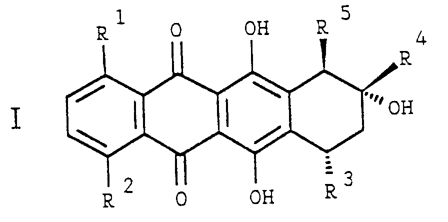 für welche gilt
R¹ ist Wasserstoff oder eine Hydroxygruppe,
R² ist Wasserstoff, eine Hydroxygruppe oder eine Alkyloxygruppe (C1-C4),
R³ ist Wasserstoff, eine Hydroxygruppe oder eine Struktur der Formel II, 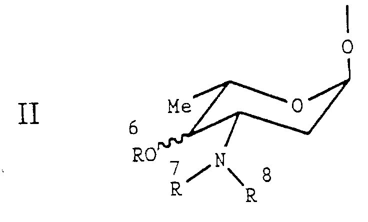
R⁴ ist CH₂CH₃, COCH₃, COCH₂OH, CHOHCH₃ oder CHOHCH₂OH,
R⁵ ist Wasserstoff, eine Hydroxygruppe, eine Methoxy carbonylgruppe oder eine Struktur der Formel II, wobei entweder R³ oder R⁵ oder beide eine Struktur der Formel II sein muß,
R⁶ ist Wasserstoff, eine Alkylgruppe (C1-C4), eine Allylgruppe, eine Benzylgruppe oder mono- oder di-methyl-oxy-substituierte Benzylgruppe, eine Tetrahydropyranylgruppe,
R⁷ ist Wasserstoff, eine Alkylgruppe (C1-C4), eine Allylgruppe, eine Benzylgruppe oder mono- oder di-methyl-oxy-substituierte Benzylgruppe und
R⁸ ist eine Struktur der Formel III oder IV, 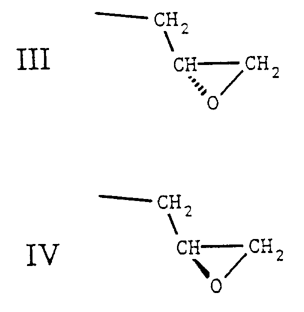
dadurch gekennzeichnet,
daß man ein Anthracyclin-Derivat der Struktur I, in der
R¹, R² und R⁴ wie oben definiert ist und
R³ ist Wasserstoff, eine Hydroxygruppe oder eine Struktur der Formel V, 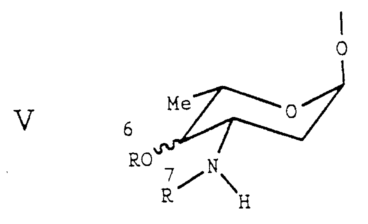
R⁵ ist Wasserstoff, eine Hydroxygruppe, eine Methoxy carbonylgruppe oder eine Struktur der Formel V, wobei entweder R³ oder R⁵ oder beide eine Struktur der Formel V sein muß, und
R⁶ und R⁷ wie oben definiert ist,
mit einem (R)- oder (S)-Glycidyl-sulfonat umsetzt und das Produkt gewinnt.

2. Verfahren nach Anspruch 1, dadurch gekennzeichnet, daß als Glycidyl-sulfonat (R)- oder (S)-Glycidyl-tosylat, (R)- oder (S)-Glycidyl-mesylat, (R)- oder (S) Glycidyl-brosylat oder (R)- oder (S)-Glycidyl-trifluormethansulfonat verwendet wird.

3. Verfahren nach Anspruch 1, dadurch gekennzeichnet, daß man zur Umsetzung Glycidyl-sulfonat in Gegenwart einer Base in einem geeigneten organischen Lösungsmittel oder Lösungsmittelgemisch bei 20°C bis zur Rückflußtemperatur des Lösungsmittels 1 bis 48 Stunden rührt und die Reaktionslösung aufarbeitet.

4. Verfahren nach Anspruch 1, dadurch gekennzeichnet, daß man bei der Aufarbeitung die Reaktionslösung mit Wasser mischt, gegebenenfalls mit einer organischen Carbonsäure neutralisiert und mit einem halogenhaltigen Lösungsmittel, wie z. B. Chloroform, Dichlormethan oder Dichlorethan extrahiert, woraufhin man die Lösung am Hochvakuum bei niedriger Temperatur einengt und durch chromatographische Trennung das Produkt erhält.

5. Verfahren nach Anspruch 1, dadurch gekennzeichnet, daß man bei der Reinigung eine chromatographische Trennung an vorbehandeltem Kieselgel durchführt, wobei das Kieselgel durch Behandeln mit einer wässrigen Pufferlösung desaktiviert ist.

6. Verfahren nach Anspruch 1, dadurch gekennzeichnet, daß die Reinigung in der Weise erfolgt, daß man eine chromatographische Grobtrennung an vorbehandeltem Kieselgel (desaktiviert durch Rühren mit wässrigem Triethylamin / Phosphat-Puffer pH 3 und anschliessendem Trockensaugen) durchführt, woraufhin man die Feintrennung durch Mitteldruckchromatographie an ebenfalls vorbehandeltem Kieselgel (desaktiviert durch Rühren mit wässrigem Triethylamin / Phosphat-Puffer pH 3 und anschliessendem Trockensaugen) anschliessen läßt.

7. Verfahren nach Anspruch 1, dadurch gekennzeichnet, daß die Aufarbeitung und die Reinigung in der Weise erfolgen, daß man die Reaktionslösung mit Wasser mischt, gegebenenfalls mit einer organischen Carbonsäure neutralisiert und mit einem halogenhaltigen Lösungsmittel, wie z. B. Chloroform, Dichlormethan oder Dichlorethan extrahiert, woraufhin man die Lösung am Hochvakuum bei niedriger Temperatur einengt und eine chromatographische Grobtrennung an vorbehandeltem Kieselgel (desaktiviert durch Rühren mit wässrigem Triethylamin / Phosphat-Puffer pH 3 und anschliessendem Trockensaugen) durchführt, woraufhin man eine oder mehrere Feintrennungen durch Mitteldruckchromatographie an ebenfalls vorbehandeltem Kieselgel (desaktiviert durch Rühren mit wässrigem Triethylamin / Phosphat-Puffer pH 3 und anschliessendem Trockensaugen) anschliessen läßt, wobei Laufmittelgemische, die zum Beispiel Toluol, Isopropanol, Acetonitril oder Dichlormethan enthalten können, zur Anwendung kommen.

8. Verfahren nach Anspruch 1, dadurch gekennzeichnet, daß eine Verbindung der Formel I hergestellt wird, für welche gilt
R¹ ist Wasserstoff oder eine Hydroxygruppe,
R² ist Wasserstoff, eine Hydroxygruppe oder eine Methoxygruppe
R³ ist eine Struktur der Formel II,
R⁴ ist CH₂CH₃, COCH₃, COCH₂OH, CHOHCH₃ oder CHOHCH₂OH,
R⁵ ist Wasserstoff, eine Hydroxygruppe, eine Methoxy carbonylgruppe oder eine Struktur der Formel II,
R⁶ ist Wasserstoff, eine Alkylgruppe (C1-C4), eine Allylgruppe, eine Benzylgruppe oder mono- oder di-methyl-oxy-substituierte Benzylgruppe, eine Tetrahydropyranylgruppe,
R⁷ ist Wasserstoff, eine Alkylgruppe (C1-C4), eine Allylgruppe, eine Benzylgruppe oder mono- oder di-methyl-oxy-substituierte Benzylgruppe und
R⁸ ist eine Struktur der Formel III oder IV.

9. Verfahren nach Anspruch 1, dadurch gekennzeichnet, daß eine Verbindung der Formel I hergestellt wird, für welche gilt
R¹ ist Wasserstoff oder eine Hydroxygruppe,
R² ist Wasserstoff, eine Hydroxygruppe oder eine Methoxygruppe,
R³ ist eine Struktur der Formel II,
R⁴ ist CH₂CH₃, COCH₃, COCH₂OH, CHOHCH₃ oder CHOHCH₂OH,
R⁵ ist Wasserstoff, eine Hydroxygruppe, eine Methoxycarbonylgruppe,
R⁶ ist Wasserstoff, eine Alkylgruppe (C1-C4), eine Allylgruppe, eine Benzylgruppe oder mono- oder di-methyl-oxy-substituierte Benzylgruppe, eine Tetrahydropyranylgruppe,
R⁷ ist Wasserstoff, eine Alkylgruppe (C1-C4), eine Allylgruppe, eine Benzylgruppe oder mono- oder di-methyl-oxy-substituierte Benzylgruppe und
R⁸ ist eine Struktur der Formel III oder IV.

10. Verfahren nach Anspruch 1, dadurch gekennzeichnet, daß eine Verbindung der Formel I hergestellt wird für welche gilt
R¹ ist Wasserstoff,
R² ist Wasserstoff, eine Hydroxygruppe oder eine Methoxygruppe,
R³ ist eine Struktur der Formel II,
R⁴ ist CH₂CH₃, COCH₃, COCH₂OH, CHOHCH₃ oder CHOHCH₂OH,
R⁵ ist Wasserstoff, eine Hydroxygruppe, eine Methoxy carbonylgruppe,
R⁶ ist Wasserstoff, eine Alkylgruppe (C1-C4), eine Allylgruppe, eine Benzylgruppe oder mono- oder di-methyl-oxy-substituierte Benzylgruppe, eine Tetrahydropyranylgruppe,
R⁷ ist Wasserstoff, eine Alkylgruppe (C1-C4), eine Allylgruppe, eine Benzylgruppe oder mono- oder di-methyl-oxy-substituierte Benzylgruppe und
R⁸ ist eine Struktur der Formel III oder IV.

11. Verfahren nach Anspruch 1, dadurch gekennzeichnet, daß eine Verbindung der Formel I hergestellt wird für welche gilt
R¹ ist Wasserstoff,
R² ist Wasserstoff, eine Hydroxygruppe oder eine Methoxygruppe,
R³ ist eine Struktur der Formel II,
R⁴ ist CH₂CH₃, COCH₃, COCH₂OH, CHOHCH₃ oder CHOHCH₂OH,
R⁵ ist Wasserstoff, eine Hydroxygruppe, eine Methoxycarbonylgruppe,
R⁶ ist Wasserstoff, eine Tetrahydropyranylgruppe,
R⁷ ist Wasserstoff, eine Alkylgruppe (C1-C4), eine Allylgruppe, eine Benzylgruppe oder mono- oder di-methyl-oxy-substituierte Benzylgruppe und
R⁸ ist eine Struktur der Formel III oder IV.

12. Verfahren nach Anspruch 1, dadurch gekennzeichnet, daß eine Verbindung der Formel I hergestellt wird, für welche gilt
R¹ ist Wasserstoff,
R² ist Wasserstoff, eine Hydroxygruppe oder eine Methoxygruppe,
R³ ist eine Struktur der Formel II,
R⁴ ist CH₂CH₃, COCH₃ COCH₂OH, CHOHCH₃ oder CHOHCH₂OH,
R⁵ ist Wasserstoff, eine Hydroxygruppe, eine Methoxycarbonylgruppe
R⁶ ist Wasserstoff, eine Tetrahydropyranylgruppe,
R⁷ ist Wasserstoff, eine Alkylgruppe (C1-C4), eine Allylgruppe und
R⁸ ist eine Struktur der Formel III oder IV.

13. Verfahren nach Anspruch 1 , dadurch gekennzeichnet, daß eine Verbindung der Formel I hergestellt wird für welche gilt
R¹ ist Wasserstoff,
R² ist eine Hydroxygruppe oder eine Methoxygruppe
R³ ist eine Struktur der Formel II,
R⁴ ist CH₂CH₃, COCH₃ oder COCH₂OH
R⁵ ist Wasserstoff, eine Hydroxygruppe,
R⁶ ist Wasserstoff,
R⁷ ist Wasserstoff, eine Alkylgruppe (C1-C4), eine Allylgruppe und
R⁸ ist eine Struktur der Formel III oder IV.

14. Verfahren nach Anspruch 1, dadurch gekennzeichnet, daß eine Verbindung der Formel I hergestellt wird für welche gilt
R¹ ist Wasserstoff,
R² ist eine Hydroxygruppe oder eine Methoxygruppe
R³ ist eine Struktur der Formel II,
R⁴ ist CH₂CH₃, COCH3 oder COCH2OH
R⁵ ist Wasserstoff, eine Hydroxygruppe,
R⁶ ist Wasserstoff,
R⁷ ist Wasserstoff, eine Alkylgruppe (C1-C4), eine Allylgruppe und
R⁸ ist eine Struktur der Formel III oder IV.

15. Verfahren nach Anspruch 1, dadurch gekennzeichnet, daß eine Verbindung der Formel I für welche gilt
R¹ ist Wasserstoff,
R² ist eine Hydroxygruppe,
R³ ist eine Struktur der Formel II,
R⁴ ist CH₂CH₃,
R⁵ ist eine Hydroxygruppe,
R⁶ ist Wasserstoff,
R⁷ ist eine Methylgruppe und
R⁸ ist eine Struktur der Formel III oder IV.

16. Verfahren nach Anspruch 1, dadurch gekennzeichnet, daß eine Verbindung der Formel I hergestellt wird für welche gilt
R¹ ist Wasserstoff,
R² ist eine Hydroxygruppe,
R³ ist eine Struktur der Formel II,
R⁴ ist CH₂CH₃,
R⁵ ist eine Hydroxygruppe,
R⁶ ist Wasserstoff,
R⁷ ist eine Methylgruppe und
R⁸ ist eine Struktur der Formel III.

## Claims

1. A process for the preparation of N-(R)-glycidylanthracyclines and N-(S)-glycidylanthracyclines of the formula I 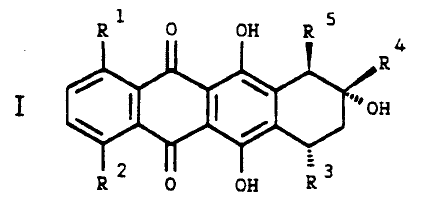 in which
R¹ is hydrogen or a hydroxyl group,
R² is hydrogen, a hydroxyl group or an alkyloxy group (C₁-C₄),
R³ is hydrogen, a hydroxyl group or a structure of the formula II 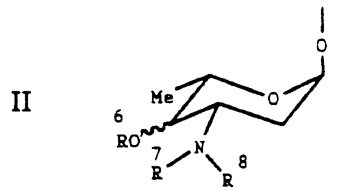
R⁴ is CH₂CH₃, COCH₃, COCH₂OH, CHOHCH₃ or CHOHCH₂OH,
R⁵ is hydrogen, a hydroxyl group, a methoxycarbonyl group or a structure of the formula II, where either R³ or R⁵ or both must be a structure of the formula II,
R⁶ is hydrogen, an alkyl group (C₁-C₄), an allyl group, a benzyl group or mono- or di-methyloxy-substituted benzyl group, a tetrahydropyranyl group,
R⁷ is hydrogen, an alkyl group (C₁-C₄), an allyl group, a benzyl group or mono- or di-methyloxy-substituted benzyl group and
R⁸ is a structure of the formula III or IV 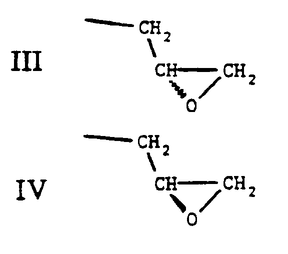
which comprises reacting an anthracycline derivative of the structure I in which
R¹, R² and R⁴ are as defined above, and
R³ is hydrogen, a hydroxyl group or a structure of the formula V 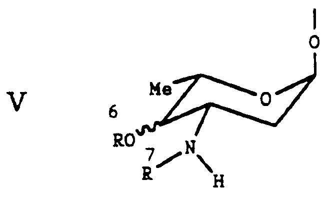
R⁵ is hydrogen, a hydroxyl group, a methoxycarbonyl group or a structure of the formula V, where either R³ or R⁵ or both must be a structure of the formula V, and
R⁶ and R⁷ are as defined above,
with an (R)- or (S)-glycidyl sulfonate, and isolating the product.

2. The process as claimed in claim 1, wherein (R)- or (S)-glycidyl tosylate, (R)- or (S)-glycidyl mesylate, (R)- or (S)-glycidyl brosylate or (R)- or (S)-glycidyl trifluoromethanesulfonate is used as glycidyl sulfonate.

3. The process as claimed in claim 1, wherein, for the reaction, glycidyl sulfonate is stirred in the presence of a base in a suitable organic solvent or solvent mixture at 20EC to the reflux temperature of the solvent for 1 to 48 hours, and the reaction solution is worked up.

4. The process as claimed in claim 1, wherein the working up entails the reaction solution being mixed with water, where appropriate being neutralized with an organic carboxylic acid and extracted with a halogen-containing solvent, such as, for example, chloroform, dichloromethane or dichloroethane, after which the solution is concentrated under high vacuum at low temperature, and the product is obtained by chromatographic separation.

5. The process as claimed in claim 1, wherein the purification entails a chromatographic separation on pretreated silica gel, where the silica gel is inactivated by treatment with an aqueous buffer solution.

6. The process as claimed in claim 1, wherein the purification is carried out in such a way that a preliminary chromatographic separation is carried out on pretreated silica gel (inactivated by stirring with aqueous triethylamine/phosphate buffer pH 3 and subsequent sucking dry), after which the final separation by medium pressure chromatography on likewise pretreated silica gel (inactivated by stirring with aqueous triethylamine/phosphate buffer pH 3 and subsequent sucking dry) follows.

7. The process as claimed in claim 1, wherein the working up and the purification are carried out in such a way that the reaction solution is mixed with water, where appropriate neutralized with an organic carboxylic acid and extracted with a halogen-containing solvent, such as, for example, chloroform, dichloromethane or dichloroethane, after which the solution is concentrated under high vacuum at low temperature, and a preliminary chromatographic separation is carried out on pretreated silica gel (inactivated by stirring with aqueous triethylamine/phosphate buffer pH 3 and subsequent sucking dry), after which one or more final separations by medium pressure chromatography on likewise pretreated silica gel (inactivated by stirring with aqueous triethylamine/phosphate buffer pH 3 and subsequent sucking dry) follow, using mobile phase mixtures which can contain, for example, toluene, isopropanol, acetonitrile or dichloromethane.

8. The process as claimed in claim 1, in which is prepared a compound of the formula I in which
R¹ is hydrogen or a hydroxyl group,
R² is hydrogen, a hydroxyl group or a methoxy group,
R³ is a structure of the formula II,
R⁴ is CH₂CH₃, COCH₃, COCH₂OH, CHOHCH₃ or CHOHCH₂OH,
R⁵ is hydrogen, a hydroxyl group, a methoxycarbonyl group or a structure of the formula II,
R⁶ is hydrogen, an alkyl group (C₁-C₄), an allyl group, a benzyl group or mono- or di-methyloxy-substituted benzyl group, a tetrahydropyranyl group,
R⁷ is hydrogen, an alkyl group (C₁-C₄), an allyl group, a benzyl group or mono- or di-methyloxy-substituted benzyl group and
R⁸ is a structure of the formula III or IV.

9. The process as claimed in claim 1, in which is prepared a compound of the formula I in which
R¹ is hydrogen or a hydroxyl group,
R² is hydrogen, a hydroxyl group or a methoxy group,
R³ is a structure of the formula II,
R⁴ is CH₂CH₃, COCH₃, COCH₂OH, CHOHCH₃ or CHOHCH₂OH,
R⁵ is hydrogen, a hydroxyl group, a methoxycarbonyl group,
R⁶ is hydrogen, an alkyl group (C₁-C₄), an allyl group, a benzyl group or mono- or di-methyloxy-substituted benzyl group, a tetrahydropyranyl group,
R⁷ is hydrogen, an alkyl group (C₁-C₄), an allyl group, a benzyl group or mono- or di-methyloxy-substituted benzyl group and
R⁸ is a structure of the formula III or IV.

10. The process as claimed in claim 1, in which is prepared a compound of the formula I in which
R¹ is hydrogen,
R² is hydrogen, a hydroxyl group or a methoxy group,
R³ is a structure of the formula II,
R⁴ is CH₂CH₃, COCH₃, COCH₂OH, CHOHCH₃ or CHOHCH₂OH,
R⁵ is hydrogen, a hydroxyl group, a methoxycarbonyl group,
R⁶ is hydrogen, an alkyl group (C₁-C₄), an allyl group, a benzyl group or mono- or di-methyloxy-substituted benzyl group, a tetrahydropyranyl group,
R⁷ is hydrogen, an alkyl group (C₁-C₄), an allyl group, a benzyl group or mono- or di-methyloxy-substituted benzyl group and
R⁸ is a structure of the formula III or IV.

11. The process as claimed in claim 1, in which is prepared a compound of the formula I in which
R¹ is hydrogen,
R² is hydrogen, a hydroxyl group or a methoxy group,
R³ is a structure of the formula II,
R⁴ is CH₂CH₃, COCH₃, COCH₂OH, CHOHCH₃ or CHOHCH₂OH,
R⁵ is hydrogen, a hydroxyl group, a methoxycarbonyl group,
R⁶ is hydrogen, a tetrahydropyranyl group,
R⁷ is hydrogen, an alkyl group (C₁-C₄), an allyl group, a benzyl group or mono- or di-methyloxy-substituted benzyl group and
R⁸ is a structure of the formula III or IV.

12. The process as claimed in claim 1, in which is prepared a compound of the formula I in which
R¹ is hydrogen,
R² is hydrogen, a hydroxyl group or a methoxy group,
R³ is a structure of the formula II,
R⁴ is CH₂CH₃, COCH₃, COCH₂OH, CHOHCH₃ or CHOHCH₂OH,
R⁵ is hydrogen, a hydroxyl group, a methoxycarbonyl group,
R⁶ is hydrogen, a tetrahydropyranyl group,
R⁷ is hydrogen, an alkyl group (C₁-C₄), an allyl group and
R⁸ is a structure of the formula III or IV.

13. The process as claimed in claim 1, in which is prepared a compound of the formula I in which
R¹ is hydrogen,
R² is a hydroxyl group or a methoxy group,
R³ is a structure of the formula II,
R⁴ is CH₂CH₃, COCH₃ or COCH₂OH,
R⁵ is hydrogen, a hydroxyl group,
R⁶ is hydrogen,
R⁷ is hydrogen, an alkyl group (C₁-C₄), an allyl group and
R⁸ is a structure of the formula III or IV.

14. The process as claimed in claim 1, in which is prepared a compound of the formula I in which
R¹ is hydrogen,
R² is a hydroxyl group or a methoxy group,
R³ is a structure of the formula II,
R⁴ is CH₂CH₃, COCH₃ or COCH₂OH,
R⁵ is hydrogen, a hydroxyl group,
R⁶ is hydrogen,
R⁷ is hydrogen, an alkyl group (C₁-C₄), an allyl group and
R⁸ is a structure of the formula III or IV.

15. The process as claimed in claim 1, in which is prepared a compound of the formula I in which
R¹ is hydrogen,
R² is a hydroxyl group,
R³ is a structure of the formula II,
R⁴ is CH₂CH₃,
R⁵ is a hydroxyl group,
R⁶ is hydrogen,
R⁷ is a methyl group and
R⁸ is a structure of the formula III or IV.

16. The process as claimed in claim 1, in which is prepared a compound of the formula I in which
R¹ is hydrogen,
R² is a hydroxyl group,
R³ is a structure of the formula II,
R⁴ is CH₂CH₃,
R⁵ is a hydroxyl group,
R⁶ is hydrogen,
R⁷ is a methyl group and
R⁸ is a structure of the formula III.

## Revendications

1. Procédé pour la préparation de N-(R)-glycidylanthracyclines et N-(S)-glycidylanthracyclines de formule I 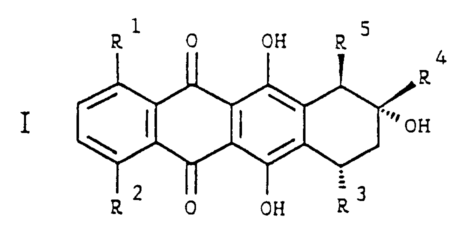 dans laquelle
R¹ est un atome d'hydrogène ou le groupe hydroxyle,
R² est un atome d'hydrogène, le groupe hydroxyle ou un groupe alkyloxy en C₁-C₄,
R³ est un atome d'hydrogène, le groupe hydroxyle ou une structure de formule II, 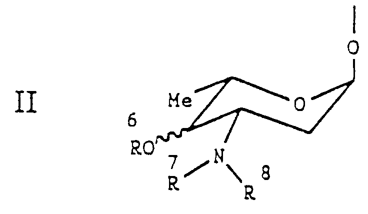
R⁴ est CH₂CH₃, COCH₃, COCH₂OH, CHOHCH₃ ou CHOHCH₂OH,
R⁵ est un atome d'hydrogène, le groupe hydroxyle, le groupe méthoxycarbonyle ou une structure de formule II, l'un ou l'autre des radicaux R³ et R⁵ ou les deux devant être une structure de formule II,
R⁶ est un atome d'hydrogène, un groupe alkyle en C₁-C₄, le groupe allyle, le groupe benzyle ou un groupe benzyle mono- ou disubstitué par le groupe méthyloxy, ou représente le groupe tétrahydropyrannyle,
R⁷ est un atome d'hydrogène, un groupe alkyle en C₁-C₄, le groupe allyle, le groupe benzyle ou un groupe benzyle mono- ou disubstitué par le groupe méthyloxy, et
R⁸ est une structure de formule III ou IV, 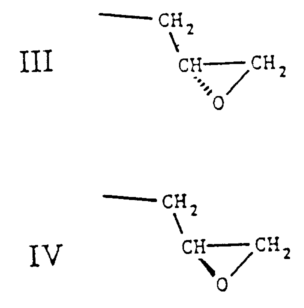
caractérisé en ce que l'on fait réagir avec un (R)- ou (S)-sulfonate de glycidyle un dérivé d'anthracycline de structure I, dans laquelle
R¹, R² et R⁴ sont tels que définis ci-dessus et
R³ est un atome d'hydrogène, le groupe hydroxyle ou une structure de formule V, 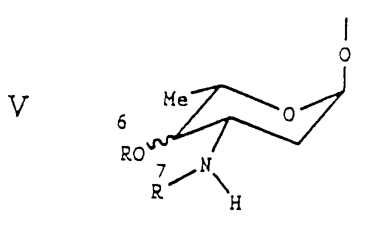
R⁵ est un atome d'hydrogène, le groupe hydroxyle, le groupe méthoxycarbonyle ou une structure de formule V, l'un ou l'autre des radicaux R³ et R⁵ ou les deux devant être une structure de formule V, et
R⁶ et R⁷ sont tels que définis ci-dessus,
et on recueille le produit.

2. Procédé selon la revendication 1, caractérisé en ce que l'on utilise comme sulfonate de glycidyle le (R)- ou (S)-tosylate de glycidyle, le (R)- ou (S)-mésylate de glycidyle, le (R)- ou (S)-brosylate de glycidyle ou le (R)- ou (S)-trifluorométhanesulfonate de glycidyle.

3. Procédé selon la revendication 1, caractérisé en ce que, dans la réaction, on agite pendant 1 à 48 heures du sulfonate de glycidyle en présence d'une base, dans un solvant organique ou mélange de solvants organiques approprié, à une température dans la plage allant de 20°C à la température de reflux du solvant, et on achève le traitement de la solution réactionnelle.

4. Procédé selon la revendication 1, caractérisé en ce que, lors du traitement final, on mélange la solution réactionnelle avec de l'eau, éventuellement on neutralise ce mélange à l'aide d'un acide carboxylique organique et on l'extrait avec un solvant halogéné, comme par exemple le chloroforme, le dichlorométhane ou le dichloréthane, à la suite de quoi on concentre la solution sous vide poussé, à basse température, et on obtient le produit par séparation chromatographique.

5. Procédé selon la revendication 1, caractérisé en ce que, lors de la purification, on effectue une séparation chromatographique sur du gel de silice prétraité, le gel de silice étant inactivé par traitement par une solution aqueuse tampon.

6. Procédé selon la revendication 1, caractérisé en ce que l'on effectue la purification en exécutant une séparation chromatographique sommaire sur du gel de silice prétraité (inactivé par agitation avec une solution aqueuse de triéthylamine / du tampon phosphate pH 3 et séparation subséquente par filtration à sec), à la suite de quoi la séparation fine peut être effectuée par chromatographie à moyenne pression sur du gel de silice également prétraité (inactivé par agitation avec une solution aqueuse de triéthylamine / du tampon phosphate pH 3 et séparation subséquente par filtration à sec).

7. Procédé selon la revendication 1, caractérisé en ce que l'on effectue le traitement final et la purification en mélangeant la solution réactionnelle avec de l'eau, et en neutralisant éventuellement ce mélange à l'aide d'un acide carboxylique organique, puis en l'extrayant avec un solvant halogéné, comme par exemple le chloroforme, le dichlorométhane ou le dichloréthane, à la suite de quoi on concentre la solution sous vide poussé, à basse température, et on effectue une séparation chromatographique sommaire sur du gel de silice prétraité (inactivé par agitation avec une solution aqueuse de triéthylamine / du tampon phosphate pH 3 et séparation subséquente par filtration à sec), à la suite de quoi on peut effectuer une ou plusieurs séparations fines par chromatographie à moyenne pression sur du gel de silice également prétraité (inactivé par agitation avec une solution aqueuse de triéthylamine / du tampon phosphate pH 3 et séparation subséquente par filtration à sec), en utilisant des mélanges d'éluants qui peuvent contenir, par exemple, du toluène, de l'isopropanol, de l'acétonitrile ou du dichlorométhane.

8. Procédé selon la revendication 1, caractérisé en ce que l'on prépare un composé de formule I dans laquelle
R¹ est un atome d'hydrogène ou le groupe hydroxyle,
R² est un atome d'hydrogène, le groupe hydroxyle ou le groupe méthoxy,
R³ est une structure de formule II,
R⁴ est CH₂CH₃, COCH₃, COCH₂OH, CHOHCH₃ ou CHOHCH₂OH,
R⁵ est un atome d'hydrogène, le groupe hydroxyle, le groupe méthoxycarbonyle ou une structure de formule II,
R⁶ est un atome d'hydrogène, un groupe alkyle en C₁-C₄, le groupe allyle, le groupe benzyle ou un groupe benzyle mono- ou disubstitué par le groupe méthyloxy, ou représente le groupe tétrahydropyrannyle,
R⁷ est un atome d'hydrogène, un groupe alkyle en C₁-C₄, le groupe allyle, le groupe benzyle ou un groupe benzyle mono- ou disubstitué par le groupe méthyloxy, et
R⁸ est une structure de formule III ou IV.

9. Procédé selon la revendication 1, caractérisé en ce que l'on prépare un composé de formule I dans laquelle
R¹ est un atome d'hydrogène ou le groupe hydroxyle,
R² est un atome d'hydrogène, le groupe hydroxyle ou le groupe méthoxy,
R³ est une structure de formule II,
R⁴ est CH₂CH₃, COCH₃, COCH₂OH, CHOHCH₃ ou CHOHCH₂OH,
R⁵ est un atome d'hydrogène, le groupe hydroxyle ou le groupe méthoxycarbonyle,
R⁶ est un atome d'hydrogène, un groupe alkyle en C₁-C₄, le groupe allyle, le groupe benzyle ou un groupe benzyle mono- ou disubstitué par le groupe méthyloxy, ou représente le groupe tétrahydropyrannyle,
R⁷ est un atome d'hydrogène, un groupe alkyle en C₁-C₄, le groupe allyle, le groupe benzyle ou un groupe benzyle mono- ou disubstitué par le groupe méthyloxy, et
R⁸ est une structure de formule III ou IV.

10. Procédé selon la revendication 1, caractérisé en ce que l'on prépare un composé de formule I dans laquelle
R¹ est un atome d'hydrogène,
R² est un atome d'hydrogène, le groupe hydroxyle ou le groupe méthoxy,
R³ est une structure de formule II,
R⁴ est CH₂CH₃, COCH₃, COCH₂OH, CHOHCH₃ ou CHOHCH₂OH,
R⁵ est un atome d'hydrogène, le groupe hydroxyle ou le groupe méthoxycarbonyle,
R⁶ est un atome d'hydrogène, un groupe alkyle en C₁-C₄, le groupe allyle, le groupe benzyle ou un groupe benzyle mono- ou disubstitué par le groupe méthyloxy, ou représente le groupe tétrahydropyrannyle,
R⁷ est un atome d'hydrogène, un groupe alkyle en C₁-C₄, le groupe allyle, le groupe benzyle ou un groupe benzyle mono- ou disubstitué par le groupe méthyloxy, et
R⁸ est une structure de formule III ou IV.

11. Procédé selon la revendication 1, caractérisé en ce que l'on prépare un composé de formule I dans laquelle
R¹ est un atome d'hydrogène,
R² est un atome d'hydrogène, le groupe hydroxyle ou le groupe méthoxy,
R³ est une structure de formule II,
R⁴ est CH₂CH₃, COCH₃, COCH₂OH, CHOHCH₃ ou CHOHCH₂OH,
R⁵ est un atome d'hydrogène, le groupe hydroxyle ou le groupe méthoxycarbonyle,
R⁶ est un atome d'hydrogène ou le groupe tétrahydropyrannyle,
R⁷ est un atome d'hydrogène, un groupe alkyle en C₁-C₄, le groupe allyle, le groupe benzyle ou un groupe benzyle mono- ou disubstitué par le groupe méthyloxy, et
R⁸ est une structure de formule III ou IV.

12. Procédé selon la revendication 1, caractérisé en ce que l'on prépare un composé de formule I dans laquelle
R¹ est un atome d'hydrogène,
R² est un atome d'hydrogène, le groupe hydroxyle ou le groupe méthoxy,
R³ est une structure de formule II,
R⁴ est CH₂CH₃, COCH₃, COCH₂OH, CHOHCH₃ ou CHOHCH₂OH,
R⁵ est un atome d'hydrogène, le groupe hydroxyle ou le groupe méthoxycarbonyle,
R⁶ est un atome d'hydrogène ou le groupe tétrahydropyrannyle,
R⁷ est un atome d'hydrogène, un groupe alkyle en C₁-C₄ ou le groupe allyle, et
R⁸ est une structure de formule III ou IV.

13. Procédé selon la revendication 1, caractérisé en ce que l'on prépare un composé de formule I dans laquelle
R¹ est un atome d'hydrogène,
R² est le groupe hydroxyle ou méthoxy,
R³ est une structure de formule II,
R⁴ est CH₂CH₃, COCH₃ ou COCH₂OH,
R⁵ est un atome d'hydrogène ou le groupe hydroxyle,
R⁶ est un atome d'hydrogène,
R⁷ est un atome d'hydrogène, un groupe alkyle en C₁-C₄ ou le groupe allyle, et
R⁸ est une structure de formule III ou IV.

14. Procédé selon la revendication 1, caractérisé en ce que l'on prépare un composé de formule I dans laquelle
R¹ est un atome d'hydrogène,
R² est le groupe hydroxyle ou méthoxy,
R³ est une structure de formule II,
R⁴ est CH₂CH₃, COCH₃ ou COCH₂OH,
R⁵ est un atome d'hydrogène ou le groupe hydroxyle,
R⁶ est un atome d'hydrogène,
R⁷ est un atome d'hydrogène, un groupe alkyle en C₁-C₄ ou le groupe allyle, et
R⁸ est une structure de formule III ou IV.

15. Procédé selon la revendication 1, caractérisé en ce que l'on prépare un composé de formule I dans laquelle
R¹ est un atome d'hydrogène,
R² est le groupe hydroxyle,
R³ est une structure de formule II,
R⁴ est CH₂CH₃,
R⁵ est le groupe hydroxyle,
R⁶ est un atome d'hydrogène,
R⁷ est le groupe méthyle, et
R⁸ est une structure de formule III ou IV.

16. Procédé selon la revendication 1, caractérisé en ce que l'on prépare un composé de formule I dans laquelle
R¹ est un atome d'hydrogène,
R² est le groupe hydroxyle,
R³ est une structure de formule II,
R⁴ est CH₂CH₃,
R⁵ est le groupe hydroxyle,
R⁶ est un atome d'hydrogène,
R⁷ est le groupe méthyle, et
R⁸ est une structure de formule III.
